# EUROPÄISCHE PATENTSCHRIFT

(11) **EP 0 635 247 B1**
(45) Veröffentlichungstag und Bekanntmachung des Hinweises auf die Patenterteilung: **04.11.1998**
(21) Anmeldenummer: 94105939.6
(22) Anmeldetag: 16.04.1994
(51) Int. Cl.: A61F 2/58

(54) **Ellbogenlifter**
Elbow lifting device
Dispositif de levage pour un coude

(30) Priorität: 21.07.1993 DE 4324399
(43) Veröffentlichungstag der Anmeldung: 25.01.1995
(73) Patentinhaber: Otto Bock Orthopädische Industrie Besitz- und Verwaltungs-Kommanditgesellschaft, 37115 Duderstadt (DE)
(72) Erfinder: Gammer, Peter, A-1130 Wien (AT); Broeckl, Heinz, A-1170 Wien (AT); Dietl, Hans, Dr., A-1180 Wien (AT)
(74) Vertreter: Gramm, Werner, Prof. Dipl.-Ing.

(56) Entgegenhaltungen:
- US-A- 2 537 402
- US-A- 2 626 398
- US-A- 3 107 358

## Beschreibung

Die Erfindung betrifft ein Verfahren zum Aufbringen eines Drehmomentes auf die Ellbogenachse einer Oberarmprothese zur Verschwenkung des Unterarmes mit Hilfe einer Federkraft.

Die Erfindung betrifft ferner einen Ellbogenlifter für eine Armprothese, deren Oberarmpaßteil über zumindest eine Ellbogenachse gelenkig mit einem Unterarmpaßteil verbunden ist, dessen Verschwenkung durch ein Federelement des Ellbogenlifters unterstützt wird.

Im Prothesenbau wird je nach Leistungsvermögen unterschieden zwischen aktiven und passiven Prothesen. Zur Gruppe der aktiven Prothesen zählen jene, die mit Eigenkraft oder mit Fremdkraft gesteuert werden. Dabei nimmt unter den Eigenkraft-Prothesen die Armprothese mit Kraftzug einen hervorragenden Platz ein. Der Arm mit dem Greiforgan Hand wird schwerkraftbedingt überwiegend auf Zug beansprucht. Deshalb muß nach konstruktiven Lösungen gesucht werden, die zu einer Zugentlastung führen. Bei der aktiven Armprothese wird das willkürliche Beugen des Unterarmes mittels Schulterbandagen aus elastischem Material in Bowdenzügen eingeleitet. Dies geschieht durch Verkürzen des Beugezuges, wobei die Befestigung zwischen Ober- und Unterarm an einem Ende des Zuges erfolgt, und die Kraft über die Schulter zum Oberarm des anderen Zuges eingeleitet wird.

Diese Prothesensysteme haben den Nachteil, daß durch fehlende Kompensation des Gewichtes des Unterarmes eine Ermüdung des Prothesenträgers eintritt; außerdem kann der Beugefunktionsbereich nicht optimal genutzt werden. Zur Kompensation des Gewichtes des Unterarmes wurden verschiedene technische Lösungen entwickelt. Die gebräuchlichsten eingesetzten Konstruktionen sind federunterstützende Systeme, die nahe dem Gelenkpunkt angebracht werden. Der Nachteil dieser Konstruktionen besteht darin, daß das höchste Drehmoment zur Unterstützung der Beugebewegung in der Strecklage des Unterarmes eingeleitet wird, und daß dieses Drehmoment bei der Beugung des Unteramres abnimmt. Der Drehmomentenverlauf ist somit genau entgegengesetzt den tatsächlichen Bedürfnissen. Ein weiterer Nachteil dieser vorbekannten Lösungen ist darin zu sehen, daß bei zu starker Erhöhung des Drehmomentes die Freischwingphase behindert und somit das natürliche Erscheinungsbild mit der Pendelphase des Unterarmes gestört wird.

Die US-A-2,626,398 offenbart einen Ellbogenlifter, der als in die Ellbogenachse integriertes, spannbares Federelement ausgebildet ist, das eine die Ellbogenachse konzentrisch übergreifende Hohlwelle beaufschlagt, die mit dem Unterarmpaßteil über Schienen drehfest verbunden ist. Vorgesehen ist überdies eine manuell betätigbare Freischwingphase. Im übrigen aber weist dieser Ellbogenlifter ebenfalls die vorstehend genannten Nachteile auf.

Der Erfindung liegt somit die Aufgabe zugrunde, ein Verfahren sowie einen Ellbogenlifter zu entwickeln, mit denen sich die vorgenannten Unzulänglichkeiten vermeiden lassen.

Diese Aufgabe wird ausgehend von dem eingangs beschriebenen Verfahren erfindungsgemäß dadurch gelöst, daß der Drehmomentenverlauf in Abhängigkeit vom Beugewinkel des Unterarmes im wesentlichen parabelförmig ist und seine niedrigsten Werte in der Strecklage von im wesentlichen 5° bis 25°, vorzugsweise 10° bis 20° sowie bei maximaler Beugung und sein Maximum bei etwa 90° aufweist.

Die genannte Aufgabe wird ausgehend von dem eingangs beschriebenen Ellbogenlifter erfindungsgemäß gelöst durch ein Getriebe, dessen Eingang von dem Federelement mit einer Zugkraft direkt oder indirekt beaufschlagt wird, und dessen Ausgang an dem inneren Ende eines mit einem inneren und einem äußeren Ende versehenen Zugorgangs angreift, dessen äußeres Ende an einem mit der Ellbogenachse einen Drehmomenthebel definierenden Fixpunkt des Oberarmpaßteils festlegbar ist, wobei die Kraftübertragung durch das Getriebe so erfolgt, daß der Verlauf des am Fixpunkt des Oberarmpaßteiles angreifenden Drehmomentes in Abhängigkeit vom Beugewinkel des Unterarmpaßteils im wesentlichen parabelförmig ist und seine niedrigsten Werte in der Strecklage von im wesentlichen 5° bis 25°, vorzugsweise 10° bis 20° sowie bei maximaler Beugung und sein Maximum bei etwa 90° aufweist.

Während also der Drehmomentenverlauf bei den vorbekannten Ellbogenliftern ausgehend von der Strecklage des Armes bis zum maximalen Beugewinkel des Unterarmes von einem Maximum nahezu linear abfällt, wird erfindungsgemäß ein Drehmomentenverlauf erzielt, der exakt den tatsächlichen Bedürnissen bei der Unterstützung der Beugung des Unterarmes entspricht. Dabei läßt sich bei richtiger Anpassung des Systems die Schwungphase des Oberarmstumpfes für das Vorbringen und Absenken des Unterarmes ausnutzen. Hierdurch kann sogar ein sonst für die Steuerung des Unterarmes erforderliches Zugseil entfallen, wodurch eine größere Bewegungsfreiheit des Amputierten ermöglicht wird.

In einer Weiterentwicklung des erfinderischen Konzeptes kann es zweckmäßig sein, wenn die Federkraft durch eine elektrische Verstellung in Abhängigkeit vom Beugewinkel sowie des Drehmomentenverlaufes verändert und/oder durch ein elektrisch aufgebrachtes, zusätzliches Drehmoment verstärkt wird. Vorrichtungsmäßig kann es vorteilhaft sein, wenn die Justierung des Federelementes über einen ersten, von einem Sensor gesteuerten Motor erfolgt. Zusätzlich kann es vorteilhaft sein, wenn im Bereich der Ellbogenachse ein zweiter, ein zusätzliches Drehmoment auf das Unterarmpaßteil aufbringender Motor vorgesehen ist.

Es ist vorteilhaft, wenn das Federelement hinsichtlich seiner Zugkraft justierbar ausgebildet ist, wobei diese Justierung manuell von außen vorzunehmen sein sollte.

Das Federelement ist vorzugsweise eine Spiralfeder, könnte aber auch eine Zugfeder oder ein ähnlich speicherndes Element sein.

Die Übertragung der Federkraft zwischen den einzelnen Stufen könnte grundsätzlich durch starre Elemente wie z. B. Hebelarme oder aber durch Kombinationen von Hebelarm, Drehgelenken, Verzahnungen oder dergleichen erfolgen. Vorteilhaft erscheint jedoch die Ausbildung des Getriebes als Kurvenscheibengetriebe, das insbesondere wie folgt ausgebildet sein kann:
a) Das Kurvenscheibengetriebe umfaßt zwei jeweils drehfest auf einer gemeinsamen Welle sitzende Kurvenscheiben;
b) das genannte Kraftübertragungselement ist ein biegeelastischer Riemen oder dergleichen, der mit seinem einen Ende direkt oder indirekt an dem Federelement angreift, die erste der beiden Kurvenscheiben bei gestreckter Armprothese weitgehend umschlingt und mit seinem anderen Ende an dieser ersten Kurvenscheibe an einem den Getriebeeingang definierenden Punkt befestigt ist;
c) das genannte Zugorgan ist ebenfalls biegeelastisch ausgebildet und ist mit seinem inneren Ende an einem den Getriebeausgang definierenden Punkt der zweiten Kurvenscheibe befestigt, die von dem Zugorgan bei zunehmender Beugung des Unterarmpaßteils zunehmend unmschlungen wird.

Dabei ist es zweckmäßig, wenn das von der zweiten Kurvenscheibe kommende Zugorgan um eine Umlenkrolle angenähert tangential an die untere Kontur des Oberarmpaßteils herangeführt ist. Um bei starker Überkompensation des Armes ein zu schnelles Vorschwingen zu verhindern, ist es vorteilhaft, wenn in die Umlenkrolle eine hydrodynamische Dämpfung eingebaut ist.

Weitere Merkmale der Erfindung sind Gegenstand der Unteransprüche und werden in Verbindung mit weiteren Vorteilen der Erfindung anhand zweier Ausführungsbeispiele näher erläuert.

In der Zeichnung sind zwei als Beispiele dienende Ausführungsformen der Erfindung dargestellt. Es zeigen:
- **Figur 1 -**: im Längsschnitt und zum Teil in Seitenansicht eine komplette Armprothese mit gestrecktem Unterarm;
- **Figur 2 -**: in vergrößertem Maßstab einen Ausschnitt der **Figur 1**;
- **Figur 3 -**: die Darstellung gemäß **Figur 2** jedoch mit stark angewinkeltem Unterarm;
- **Figur 4 -**: eine mit zusätzlichen Elektroantrieben ausgerüstete Ausführungsform in einer Darstellung gemäß **Figur 3** und
- **Figur 5 -**: drei Drehmomentenverläufe, aufgetragen über den Beugewinkeln des Unterarmes.

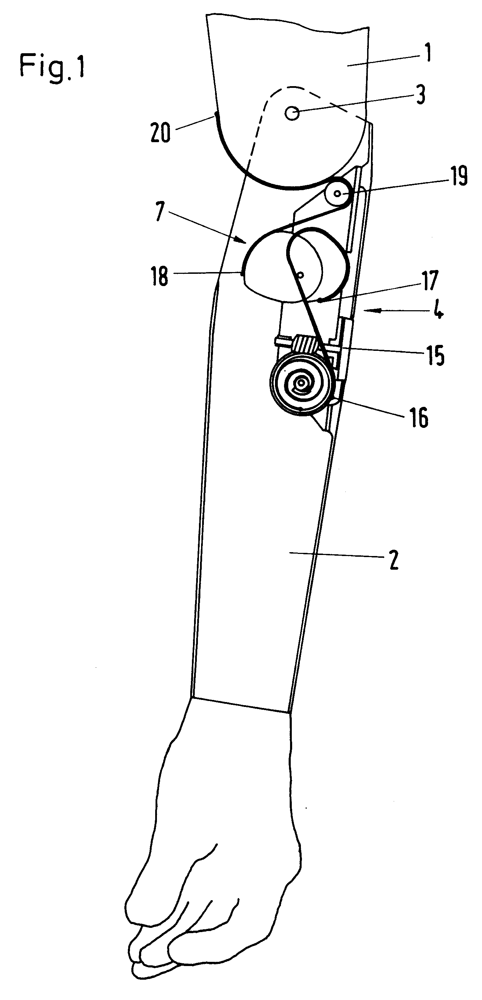

Die in Figur 1 dargestellte Armprothese umfaßt ein Oberarmpaßteil 1 sowie ein Unterarmpaßteil 2, die über eine Ellbogenachse 3 gelenkig miteinander verbunden sind. Zur Unterstützung der Verschwenkung des Unterarmpaßteils 2 in verschiedene Beugestellungen ist ein Ellbogenlifter 4 vorgesehen. Dieser weist ein alle seine Bauteile aufnehmendes bzw. umschließendes Gehäuse 5 auf, das in eine angepaßte Ausnehmung im Unterarmpaßteil 2 eingesetzt ist.

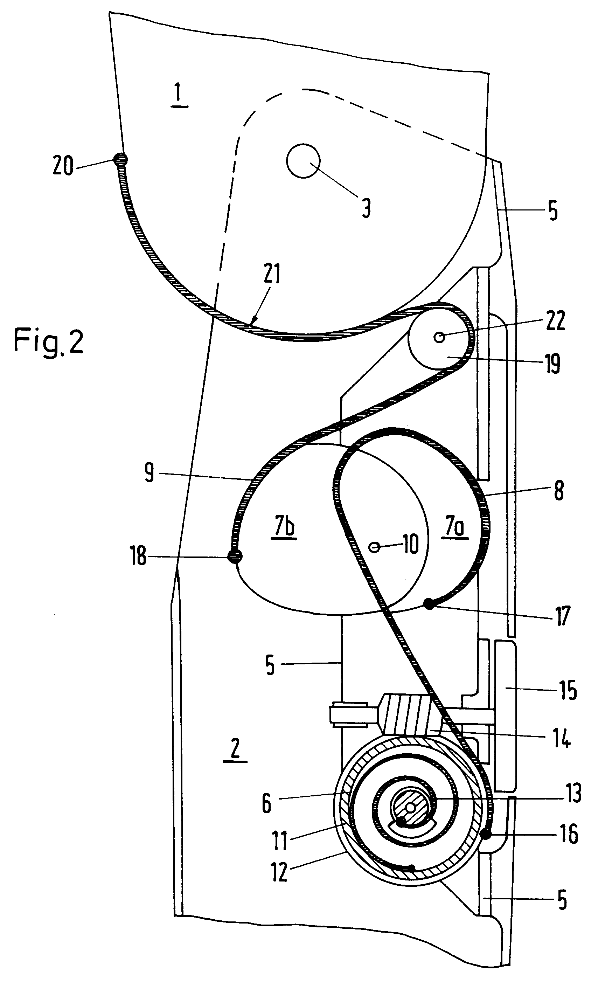

Der in Figur 2 dargestellte Ellbogenlifter 4 umfaßt im wesentlichen eine Spiralfeder 6, ein Kurvenscheibengetriebe 7, ein die Kraft von der Spiralfeder 6 auf das Kurvenscheibengetriebe 7 übertragendes, als biegeelastischer Riemen 8 ausgebildetes Kraftübertragungselement sowie ein das Kurvenscheibengetriebe 7 kinematisch mit dem Oberarmpaßteil 1 verbindendes Zugorgan 9.

Das Kurvenscheibengetriebe 7 umfaßt zwei Kurvenscheiben 7a und 7b, die jeweils drehfest auf einer gemeinsamen Welle 10 sitzen.

Die Spiralfeder 6 ist in einem Federgehäuse 11 befestigt und beaufschlagt als Drehkraft eine Abtriebsscheibe 12, die auf einer Achse 13 sitzt. Die Vorspannung der Spiralfeder 6 kann entsprechend dem auszugleichenden, am Unterarmpaßteil 2 angreifenden Gewicht verändert werden und zwar durch Verdrehung eines Schneckenrades 14, für dessen Betätigung eine von außen zugängliche, manuell betätigbare Drehscheibe 15, ein Rändelrad oder dergelichen vorgesehen ist.

Der Riemen 8 ist mit seinem einen Ende an einem Fixpunkt 16 an der Abtriebsscheibe 12 befestigt, umschlingt nahezu vollständig die äußere Kurvenkontur der ersten Kurvenscheibe 7a und ist mit seinem anderen Ende am Außenumfang dieser ersten Kurvenscheibe 7a über einen Fixpunkt 17 befestigt, der den Getriebeeingang des Kurvenscheibengetriebes 7 definiert.

Das ebenso wie der Riemen 8 biegeelastisch ausgebildete Zugorgan 9 ist mit seinem inneren Ende an einem den Getriebeausgang definierenden Fixpunkt 18 am Umfang der zweiten Kurvenscheibe 7b befestigt, ist dann um eine im Gehäuse 5 gelagerte Umlenkrolle 19 geführt und ist mit seinem äußeren, aus dem Gehäuse 5 herausgeführten Ende an einem mit der Ellbogenachse 3 einen Drehmomenthebel definierenden Fixpunkt 20 des Oberarmpaßteils 1 festgelegt. Dabei läßt Figur 2 erkennen, daß das Zugorgan 9 bei gestrecktem Unterarmpaßteil 2 die äußere Kurvenkontur der zweiten Kurvenscheibe 7b nur über einen kurzen Abschnitt umschlingt und im Bereich zwischen der Umlenkrolle 19 und dem Fixpunkt 20 am Oberarmpaßteil 1 an dessen unterer, angenähert kreissegmentförmig ausgebildeten Kontur 21 anliegt.

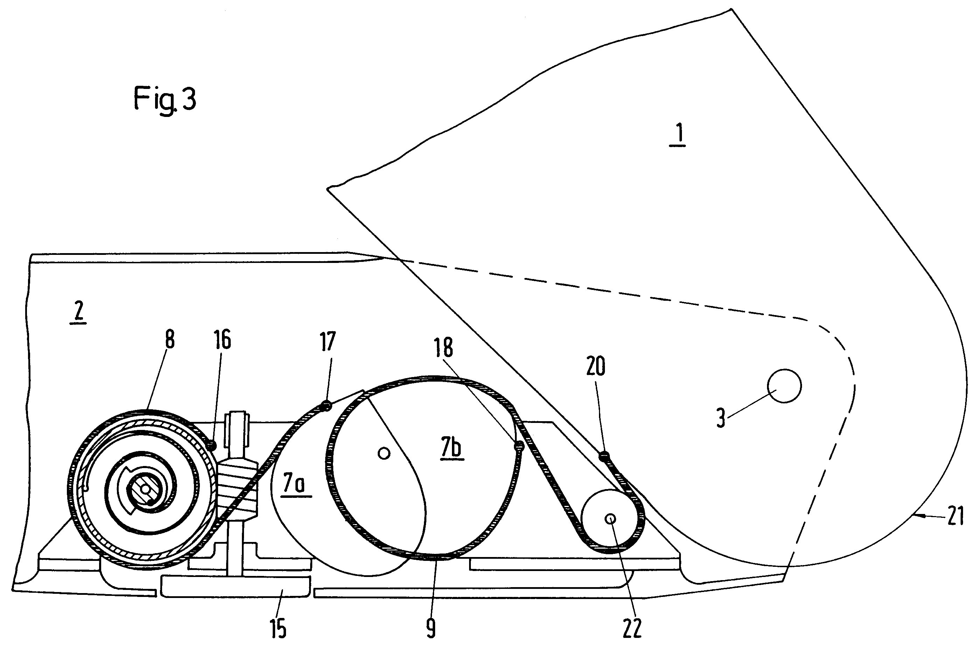

Figur 3 zeigt das Unterarmpaßteil 2 in seinem maximalen Beugewinkel. Ein Vergleich mit der Figur 2 macht deutlich, daß ausgehend aus der gestreckten Armlage mit zunehmendem Beugewinkel des Unterarmpaßteils 2 gegenüber dem Oberarmpaßteil 1 der Riemen 8 von der ersten Kurvenscheibe 7a ab- und auf die von der Spiralfeder 6 angetriebene Abtriebsscheibe 12 aufgewickelt wird, während das Zugorgan 9 sich zunehmend von der unteren Kontur 21 des Oberarmpaßteils 1 löst und auf die zweite Kurvenscheibe 7b aufgewickelt wird. Figur 3 läßt ferner erkennen, daß die Umlenkrolle 19 derart angeordnet ist, daß das Zugorgan 9 an die Kontur 21 annähernd tangential herangeführt wird.

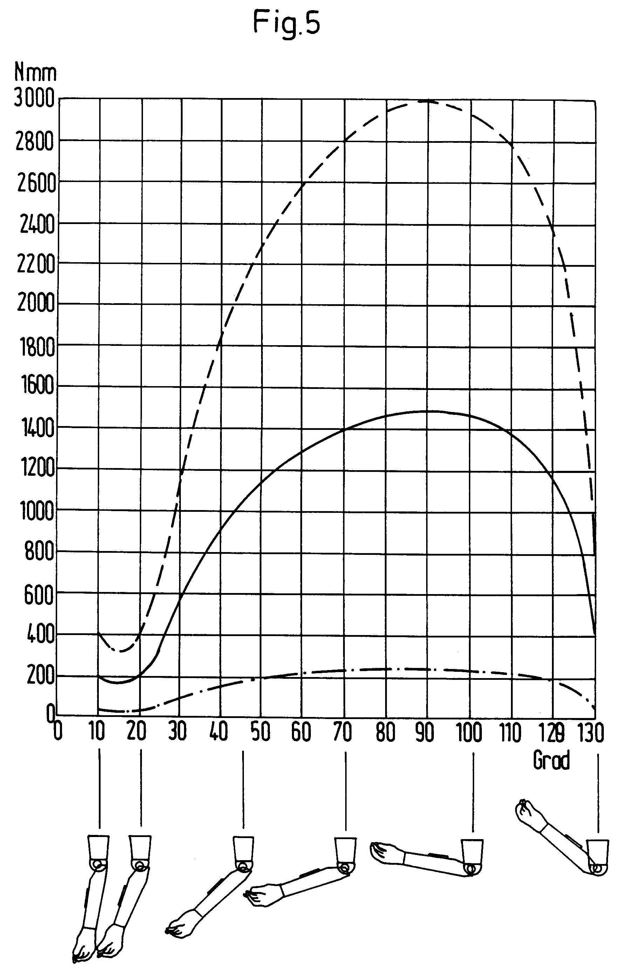

Das Getriebe 7 ist in Verbindung mit der Charakteristik des Federelementes 6 so ausgelegt, daß der Verlauf des auf die Ellbogenachse 3 aufgebrachten Drehmomentes in Abhängigkeit vom Beugewinkel des Unterarmpaßteils 2 angenähert parabelförmig ist (siehe Figur 5) und seine niedrigsten Werte in der Strecklage von etwa 10° bis 20° sowie bei maximaler Beugung und sein Maximum bei etwa 90° aufweist. Dies wird mit dem in den Figuren 2 und 3 dargestellten Ausführungsbeispielen durch eine entsprechende Gestaltung des Kurvenverlaufes der beiden Kurvenscheiben 7a, 7b sowie durch ihre Zuordnung zueinander erreicht, wodurch auch eine entsprechende Kompensation der Federkraftkennlinie vorgenommen wird. Den einstellbaren Bereich dieser Kompensation zeigen in Figur 5 die über dem Beugewinkel des Unterarmpaßteils 2 aufgetragenen Drehmomentlinien.

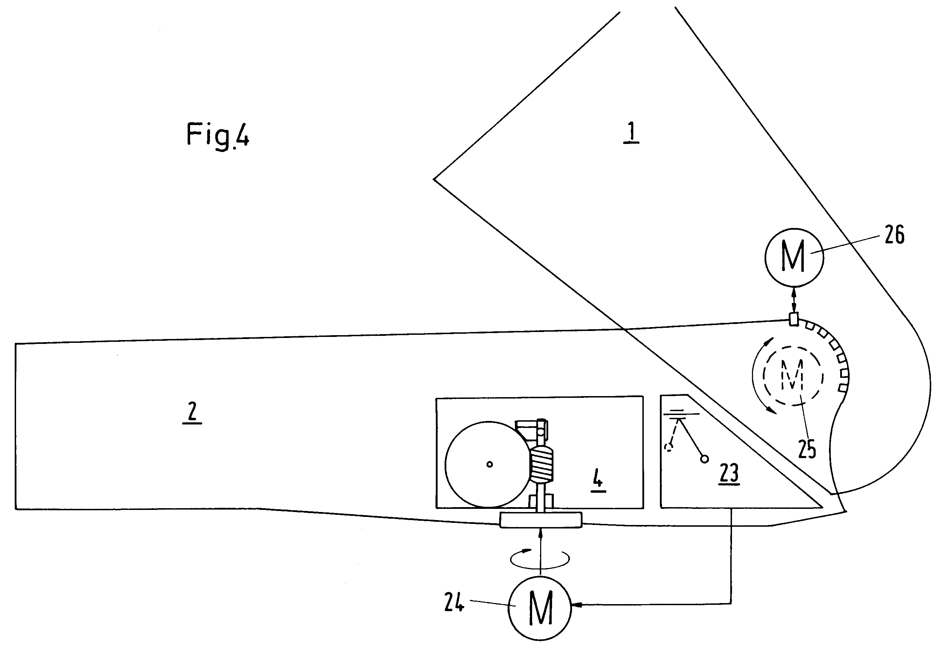

In Figur 4 ist schematisch angedeutet, daß die Justierung des Federelementes 6 auch über einen ersten, von einem Sensor 23 in Abhängigkeit von dem Beugewinkel gesteuerten Motor 24 erfolgen kann. Somit läßt sich durch eine elektrische Verstellung die Federkraft in Abhängigkeit vom Beugewinkel sowie des Drehmomentverlaufes verändern. Darüber hinaus kann im Bereich der Ellbogenachse 3 ein zweiter, ein zusätzliches Drehmoment auf das Unterarmpaßteil 2 aufbringender Motor 25 vorgesehen sein. Dadurch läßt sich die Federkraft elektrisch verstärken. Schließlich ist in Figur 4 noch ein dritter Motor 26 angedeutet, mit dem sich eine elektrische Arretierung des Unterarmpaßteils 2 in einer gewünschten Beugestellung erreichen läßt.

Eine vergleichbare, mit der durch die beiden Kurvenscheiben 7a,7b erzielte Kinematik ließe sich durch entsprechende Kurvenausbildung der Abtriebsscheibe 12, der Umlenkrolle 19 und/oder der Kontur 21 erzielen.

## Patentansprüche

1. Verfahren zum Aufbringen eines Drehmomentes auf die Ellbogenachse einer Oberarmprothese zur Verschwenkung des Unterarmes mit Hilfe einer Federkraft, **dadurch gekennzeichnet,** daß der Drehmomentenverlauf in Abhängigkeit vom Beugewinkel des Unterarmes im wesentlichen parabelförmig ist und seine niedrigsten Werte in der Strecklage von im wesentlichen 5° bis 25°, vorzugsweise 10° bis 20° sowie bei maximaler Beugung und sein Maximum bei im wesentlichen 90° aufweist.

2. Verfahren nach Anspruch 1, **dadurch gekennzeichnet**, daß die Federkraft durch eine elektrische Verstellung in Abhängigkeit vom Beugewinkel sowie des Drehmomentenverlaufes verändert und/oder durch ein elektrisch aufgebrachtes, zusätzliches Drehmoment verstärkt wird.

3. Ellbogenlifter für eine Armprothese, deren Oberarmpaßteil (1) über zumindest eine Ellbogenachse (3) gelenkig mit einem Unterarmpaßteil (2) verbunden ist, dessen Verschwenkung durch ein Federelement (6) des Ellbogenlifters unterstützt wird, **gekennzeichnet durch** ein Getriebe (7), dessen Eingang (17) von dem Federelement (6) mit einer Zugkraft beaufschlagt wird, und dessen Ausgang (18) an dem inneren Ende eines mit einem inneren und einem äußeren Ende versehenen Zugorgans (9) angreift, dessen äußeres Ende an einem mit der Ellbogenachse (3) einen Drehmomenthebel definierenden Fixpunkt (20) des Oberarmpaßteils (1) festlegbar ist, wobei die Kraftübertragung durch das Getriebe (7) so erfolgt, daß der Verlauf des am Fixpunkt (20) des Oberarmpaßteiles (1) angreifenden Drehmomentes in Abhängigkeit vom Beugewinkel des Unterarmpaßteils (2) im wesentlichen parabelförmig ist und seine niedrigsten Werte in der Strecklage von im wesentlichen 5° bis 25°, vorzugsweise 10° bis 20° sowie bei maximaler Beugung und sein Maximum bei im wesentlichen 90° aufweist.

4. Ellbogenlifter nach Anspruch 3, **gekennzeichnet durch** seine Ausbildung als einteilige Baugruppe, die in eine Ausnehmung des Unterarmpaßteils (2) einsetzbar ist.

5. Ellbogenlifter nach Anspruch 3 oder 4, **dadurch gekennzeichnet**, daß das Federelement (6) eine Spiralfeder ist.

6. Ellbogenlifter nach Anspruch 3, 4 oder 5, **dadurch gekennzeichnet**, daß das Federelement (6) hinsichtlich seiner Zugkraft justierbar ausgebildet ist.

7. Ellbogenlifter nach Anspruch 5 und 6, **dadurch gekennzeichnet**, daß eine Vorspannung des Federelementes (6) über ein Schneckenrad (14) justierbar ist.

8. Ellbogenlifter nach Anspruch 6 oder 7, **dadurch gekennzeichnet**, daß die Justierung des Federelementes (6) über einen ersten, von einem Sensor (23) gesteuerten Motor (24) erfolgt.

9. Ellbogenlifter nach einem der Ansprüche 3 bis 8, **dadurch gekennzeichnet**, daß das Federelement (6) als Drehkraft an einer Abtriebsscheibe (12) angreift, an der ein mit dem Getriebeeingang (17) in Verbindung stehendes Kraftübertragungselement (8) festgelegt ist.

10. Ellbogenlifter nach einem der Ansprüche 3 bis 9, **dadurch gekennzeichnet**, daß das Getriebe (7) ein Kurvenscheibengetriebe ist.

11. Ellbogenlifter nach Anspruch 10, **gekennzeichnet durch** folgende Merkmale:
a) Das Kurvenscheibengetriebe (7) umfaßt zwei jeweils drehfest auf einer gemeinsamen Welle (10) sitzende Kurvenscheiben (7a,7b);
b) das Kraftübertragungselement (8) ist ein biegeelastischer Riemen, der mit seinem einen Ende an dem Federelement (6) angreift, die erste (7a) der beiden Kurvenscheiben (7a,7b) bei gestreckter Armprothese weitgehend umschlingt und mit seinem anderen Ende an dieser ersten Kurvenscheibe (7a) an einem den Getriebeeingang (17) definierenden Punkt befestigt ist;
c) das Zugorgan (9) ist ebenfalls biegeelastisch ausgebildet und ist mit seinem inneren Ende an einem den Getriebeausgang (18) definierenden Punkt der zweiten Kurvenscheibe (7b) befestigt, die von dem Zugorgan (9) bei zunehmender Beugung des Unterarmpaßteils (2) zunehmend unmschlungen wird.

12. Ellbogenlifter nach Anspruch 11, **dadurch gekennzeichnet**, daß das von der zweiten Kurvenscheibe (7b) kommende Zugorgan (9) um eine Umlenkrolle (19) im wesentlichen tangential an die untere Kontur (21) des Oberarmpaßteils (1) herangeführt ist.

13. Ellbogenlifter nach Anspruch 12, **dadurch gekennzeichnet**, daß das Zugorgan (9) bei gestreckter Armprothese im Bereich zwischen der Umlenkrolle (19) und dem Fixpunkt (20) am Oberarmpaßteil (1) an dessen unterer, im wesentlichen kreissegmentförmig ausgebildeter Kontur (21) anliegt.

14. Ellbogenlifter nach einem der Ansprüche 3 bis 13, **gekennzeichnet durch** ein alle Bauteile aufnehmendes bzw. umschließendes Gehäuse (5).

15. Ellbogenlifter nach einem der Ansprüche 3 bis 14, **dadurch gekennzeichnet**, daß im Bereich der Ellbogenachse (3) ein zweiter, ein zusätzliches Drehmoment auf das Unterarmpaßteil (2) aufbringender Motor (25) vorgesehen ist.

16. Ellbogenlifter nach einem der Ansprüche 12 bis 15, **dadurch gekennzeichnet**, daß in die Umlenkrolle (19) eine hydrodynamische Dämpfung eingebaut ist.

## Claims

1. Process for the application of a torque to the elbow axis of an upper-arm prosthesis with a view to pivoting the forearm with the aid of a spring force, **characterised in that** the torque curve as a function of the bending angle of the forearm is substantially parabolic and exhibits its lowest values in the extended position of substantially 5° to 25°, preferably 10° to 20°, and also at maximal bending and exhibits its maximum at substantially 90°.

2. Process according to Claim 1, **characterised in that** the spring force is varied by means of an electrical adjustment as a function of the bending angle and also of the torque curve and/or is reinforced by means of an additional torque which is applied electrically.

3. Elbow lifter for an arm prosthesis, the upper-arm mating part (1) of which is connected in articulated manner via at least one elbow axis (3) to a forearm mating part (2), the pivoting of which is assisted by a spring element (6) pertaining to the elbow lifter, **characterised by** a gear mechanism (7), the input (17) of which is acted upon by the spring element (6) with a tensile force and the output (18) of which is applied to the inner end of a tensile member (9) provided with an inner end and an outer end, the outer end of said tensile member being capable of being secured to a fixed point (20) of the upper-arm mating part (1) defining a torque lever with the elbow axis (3), the transfer of force being effected by the gear mechanism (7) in such a way that the curve of the torque applied to the fixed point (20) of the upper-arm mating part (1) as a function of the bending angle of the forearm mating part (2) is substantially parabolic and exhibits its lowest values in the extended position of substantially 5° to 25°, preferably 10° to 20°, and also at maximal bending and exhibits its maximum at substantially 90°.

4. Elbow lifter according to Claim 3, **characterised by** its formation as a one-piece subassembly which is capable of being inserted in a recess in the forearm mating part (2).

5. Elbow lifter according to Claim 3 or 4, **characterised in that** the spring element (6) is a spiral spring.

6. Elbow lifter according to Claim 3, 4 or 5, **characterised in that** the spring element (6) is of adjustable construction with respect to its tensile force.

7. Elbow lifter according to Claims 5 and 6, **characterised in that** a bias of the spring element (6) is adjustable via a worm wheel (14).

8. Elbow lifter according to Claim 6 or 7, **characterised in that** the adjustment of the spring element (6) is effected via a first motor (24) which is controlled by a sensor (23).

9. Elbow lifter according to one of Claims 3 to 8, **characterised in that** the spring element (6) is applied by way of torsional force to a driven pulley (12), to which a force-transfer element (8) linked to the input (17) of the gear mechanism is secured.

10. Elbow lifter according to one of Claims 3 to 9, **characterised in that** the gear mechanism (7) is a disk cam mechanism.

11. Elbow lifter according to Claim 10, **characterised by** the following features:
a) the disk cam mechanism (7) comprises two cam disks (7a, 7b), each seated in torsion-resistant manner on a common shaft (10);
b) the force-transfer element (8) is a pliably resilient belt which is applied to the spring element (6) by its one end, largely wraps around the first (7a) of the two cam disks (7a, 7b) when the arm prosthesis is extended and is attached by its other end to this first cam disk (7a) at a point defining the input (17) of the gear mechanism;
c) the tensile member (9) is likewise of pliably resilient construction and is attached by its inner end to a point on the second cam disk (7b) defining the output (18) of the gear mechanism, the tensile member (9) wrapping increasingly around said second cam disk with increasing bending of the forearm mating part (2).

12. Elbow lifter according to Claim 11, **characterised in that** the tensile member (9) coming from the second cam disk (7b) is guided to the lower contour (21) of the upper-arm mating part (1) in substantially tangential manner around a deflection pulley (19).

13. Elbow lifter according to Claim 12, **characterised in that** when the arm prosthesis is extended the tensile member (9) bears in the region between the deflection pulley (19) and the fixed point (20) on the upper-arm mating part (1) against the lower contour (21) of said upper-arm mating part which has approximately the shape of a circular segment.

14. Elbow lifter according to one of Claims 3 to 13, **characterised by** a housing (5) receiving or surrounding all the components.

15. Elbow lifter according to one of Claims 3 to 14, **characterised in that** a second motor (25) applying an additional torque to the forearm mating part (2) is provided in the region of the elbow axis (3).

16. Elbow lifter according to one of Claims 12 to 15, **characterised in that** a hydrodynamic damping device is incorporated into the deflection pulley (19).

## Revendications

1. Procédé pour appliquer un moment de rotation sur l'axe cubital d'une prothèse du membre supérieur pour faire pivoter l'avant-bras à l'aide d'une force élastique, caractérisé en ce que l'allure du moment de rotation est sensiblement parabolique en fonction de l'angle de flexion de l'avant-bras et présente ses valeurs les plus faibles dans la position d'extension de sensiblement 5° à 25°, de préférence 10° à 20°, ainsi que pour la flexion maximale, et son maximum pour sensiblement 90°.

2. Procédé selon la revendication 1, caractérisé en ce que la force élastique est modifiée par un réglage électrique en fonction de l'angle de flexion ainsi que de l'allure du moment de rotation et/ou renforcé par un moment de rotation additionnel, appliqué électriquement.

3. Releveur cubital pour prothèse du membre supérieur, dont la partie adaptatrice du bras (1) est reliée de manière articulée par au moins un axe cubital (3) avec une partie adaptatrice (2) de l'avant-bras, dont le pivotement est assisté par un élément élastique (6) du releveur cubital, caractérisé par un mécanisme de transmission (7), sur l'entrée (17) duquel l'élément élastique (6) applique une force de traction, et dont la sortie (18) est en prise avec l'extrémité intérieure d'un organe de traction (9) muni d'une extrémité intérieure et d'une extrémité extérieure, laquelle peut être assujettie à un point fixe (20) de la pièce adaptatrice du bras (1), lequel point fixe (20) définit un levier avec l'axe cubital (3), la transmission de force s'effectuant par le mécanisme de transmission (7) de façon que l'allure du moment de rotation s'appliquant au point fixe (20) de la pièce adaptatrice du bras (1) soit sensiblement parabolique en fonction de l'angle de flexion de la pièce adaptatrice de l'avant bras (2) et présente ses valeurs les plus faibles dans la position d'extension de sensiblement 5° à 25°, de préférence 10° à 20° ainsi que pour la flexion maximale, et son maximum pour sensiblement 90°.

4. Releveur cubital selon la revendication 3, caractérisé par son agencement sous la forme d'un ensemble constructif d'une seule pièce pouvant être inséré dans un évidement de la pièce adaptatrice de l'avant bras (2).

5. Releveur cubital selon la revendication 3 ou 4, caractérisé en ce que l'élément élastique (6) est un ressort spirale.

6. Releveur cubital selon la revendication 3, 4 ou 5, caractérisé en ce que l'élément élastique (6) est réalisé ajustable en ce qui concerne sa force de traction.

7. Releveur cubital selon la revendication 5 ou 6, caractérisé en ce qu'une précontrainte de l'élément élastique (6) est ajustable par une roue à vis d'engrènement (14).

8. Releveur cubital selon la revendication 6 ou 7, caractérisé en ce que l'ajustement de l'élément élastique (6) se fait par l'intermédiaire d'un premier moteur (24) piloté par un détecteur (23).

9. Releveur cubital selon l'une des revendications 3 à 8, caractérisé en ce que l'élément élastique (6) est en prise d'application d'une force de rotation sur un disque mené (12), auquel est fixé un élément de transmission de force (8) qui est en liaison avec l'entrée (17) du mécanisme de transmission.

10. Releveur cubital selon l'une des revendications 3 à 9, caractérisé en ce que le mécanisme de transmission (7) est un mécanisme de transmission à cames.

11. Releveur cubital selon la revendication 10, caractérisé par les particularités suivantes :
a) le mécanisme de transmission à cames (7) comprend deux cames (7a, 7b) montées sur un arbre commun (10) avec lequel elles sont chacune liées en rotation;
b) l'élément de transmission de force (8) est une courroie élastiquement flexible qui est en prise par sa première extrémité avec l'élément élastique (6), entoure en grande partie la première (7a) des deux cames (7a, 7b) lorsque la prothèse du membre supérieur est en extension, et est fixée par son autre extrémité à cette première came (7a) en un point définissant l'entrée (17) du mécanisme de transmission;
c) l'organe de traction (9) est également réalisé élastiquement flexible et est fixé par son extrémité intérieure en un point de la deuxième came (7b), lequel point définit la sortie (18) du dispositif de transmission, la deuxième came (7b) étant entourée de manière croissante par l'organe de traction (9) lorsque la flexion de la pièce adaptatrice (2) de l'avant-bras augmente.

12. Releveur cubital selon la revendication 11, caractérisé en ce que l'organe de traction (9) venant de la deuxième came (7b) est amené autour d'un galet de renvoi (19) sensiblement tangentiellement au contour inférieur (21) de la pièce adaptatrice du bras (1).

13. Releveur cubital selon la revendication 12, caractérisé en ce que lorsque la prothèse du membre supérieur est en extension, l'organe de traction (9), dans la zone entre le galet de renvoi (19) et le point fixe (20), s'appuie sur le contour inférieur, réalisé sensiblement en forme de segment de cercle, de la pièce adaptatrice du bras (1).

14. Releveur cubital selon l'une des revendications 3 à 13, caractérisé par un boîtier (5) recevant ou enfermant tous les composants.

15. Releveur cubital selon l'une des revendications 3 à 14, caractérisé en ce qu'il est prévu dans la zone de l'axe cubital (3) un second moteur (25) appliquant un moment de rotation additionnel sur la pièce adaptatrice de l'avant-bras (2).

16. Releveur cubital selon l'une des revendications 12 à 15, caractérisé en ce qu'un amortissement hydrodynamique est installé dans le galet de renvoi (19).
